(19) Europäisches Patentamt
European Patent Office
Office européen des brevets

(11) **EP 3 108 814 A1**

(12) **EUROPEAN PATENT APPLICATION**

(43) Date of publication:
**28.12.2016 Bulletin 2016/52**

(51) Int Cl.:
*A61B 6/04* (2006.01)  *A61B 6/00* (2006.01)
*A61F 5/37* (2006.01)

(21) Application number: **16171863.0**

(22) Date of filing: **30.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.06.2015 JP 2015125229**

(71) Applicant: **Konica Minolta, Inc.**
**Tokyo 100-7015 (JP)**

(72) Inventors:
• **HOSHINO, Yoshihide**
  **Tokyo, 100-7015 (JP)**
• **NISHINO, Satoshi**
  **Tokyo, 100-7015 (JP)**

(74) Representative: **Gille Hrabal**
**Brucknerstrasse 20**
**40593 Düsseldorf (DE)**

(54) **X-RAY TALBOT IMAGING DEVICE**

(57)     To provide an X-ray Talbot imaging device (1) that includes a holding unit (20) capable of properly adjusting an angle (Θ) of a knee joint section with respect to a radiation emission direction.

The X-ray Talbot imaging device (1) includes a subject platform (13) configured to hold a subject (H), a radiation generator (11) configured to emit radiation toward the subject (H), and a holding unit (20) configured to hold a knee joint section as the subject (H) on the subject platform (13). The holding unit (20) includes a first holding section (22), a second holding section (23), a pivot fixing mechanism (B, 26), and an angle adjustment mechanism (A, 25). The first holding section (22) is configured to hold a proximal side of the joint section. The second holding section (23) is configured to hold a distal side of the joint section. The pivot fixing mechanism (B, 26) is capable of pivoting the first holding section (22) and the second holding section (23) relative to each other within a plane orthogonal to a grating surface of a phase grating (14) and capable of fixing a relative pivot angle (θ1) between the first holding section (22) and the second holding section (23). The angle adjustment mechanism (A, 25) is configured to change an angle (Θ) of the knee joint section as the subject (H) held by the first holding section (22) and the second holding section (23), with respect to a radiation emission direction.

FIG. 3

EP 3 108 814 A1

## Description

Background of the Invention

Field of the Invention

The present invention relates to an X-ray Talbot imaging device using a Talbot interferometer or a Talbot-Lau interferometer.

Description of the Related Art

An X-ray imaging device using a Talbot interferometer or a Talbot-Lau interferometer that captures a phase shift of an X-ray generated when the X-ray is transmitted through an object, and using a radiation flat panel detector (FPD) is known (for example, refer to US Patent No. 5812629 and Junji Tanaka, et al. "Cadaveric and in vivo human joint imaging based on differential phase contrast by X-ray Talbot-Lau interferometry", Z. Med. Phys. 23(2013)222-227). Hereinafter, the X-ray imaging device using a Talbot interferometer and a Talbot-Lau interferometer will be referred to as an X-ray Talbot imaging device.

The X-ray Talbot imaging device includes a phase grating (also referred to as a first grating, a G1 grating, or the like) and a second grating (also referred to as a G2 grating), each of which has slits at a predetermined interval. (The device also includes a source grating in a case where the Talbot-Lau interferometer is used). The X-ray Talbot imaging device arranges the second grating at a position where a self-image of the phase grating is formed in a predetermined period downstream of an X-ray emission direction of the phase grating by emitting an X-ray from an X-ray source to the phase grating. Then, by arranging the second grating such that an extending direction of the slit in the second grating is slightly tilted with respect to the extending direction of the phase grating, a moire fringe is formed on the second grating. Subsequently, it is configured such that the image over which the moire fringe is superposed (hereinafter, referred to as a moire image) is detected by an X-ray detector arranged downstream of the second grating and imaged.

When a subject is arranged between the X-ray source (or source grating) and the phase grating, or between the phase grating and the second grating, distortion is generated on the moire fringe due to the subject. Therefore, by imaging a plurality of moire images on the X-ray Talbot imaging device while moving the phase grating and the second grating relative to each other (fringe scanning method), and then, by performing image analysis on the moire images in image processing, it is possible to reconstruct and generate images such as a differential phase image, an absorption image, and a small-angle scattering image. Moreover, using another method (Fourier transformation method), by imaging one moire image by the X-ray Talbot imaging device while the subject exits, and by performing Fourier transformation, or the like, on the moire image in image processing, it is also possible to reconstruct and generate an image such as a differential phase image.

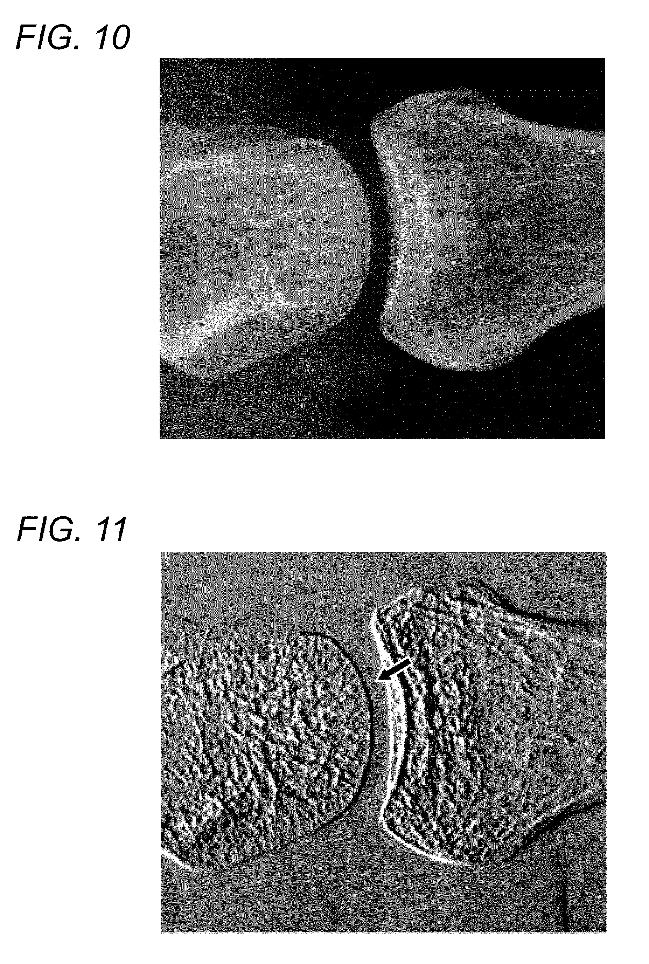

The present inventors have found in their studies that, in the differential phase image thus generated by reconstructing the moire image captured by the X-ray Talbot imaging device, it is possible to visualize, in an image as indicated by an arrow in Fig. 11, an end portion of an articular cartilage (specifically, an interface between the articular cartilage and its surrounding synovial fluid, and the same applies to the following), which cannot be imaged in a conventional absorption image (refer to Fig. 10) or the like.

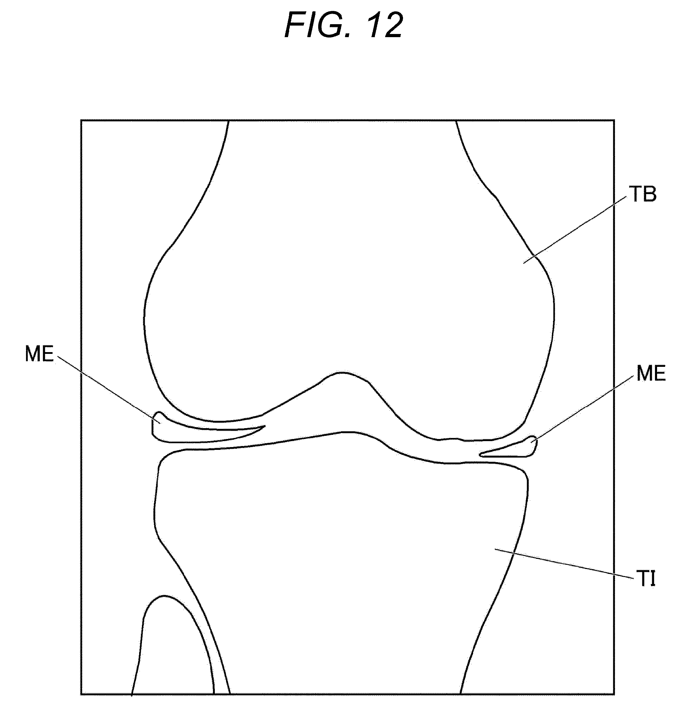

In this manner, since it is possible to image a cartilage in a differential phase image generated by reconstruction of the moire image captured by the X-ray Talbot imaging device, diagnosis of rheumatism, cartilage damage, or the like, can be performed, for example, using the differential phase image. As illustrated in Fig. 12, it is known that a cartilage-like meniscus ME between a thighbone TB and a shinbone TI in a knee joint section (to be precise, an interface between the meniscus and the cartilage of the thighbone or the shinbone) can be imaged in a differential phase image.

Accordingly, when a doctor examines the meniscus and the cartilage of the thighbone or the shinbone, imaged on the differential phase image, the doctor can properly diagnose the degree of damage or wear in the meniscus, or of damage or wear in the cartilage of the thighbone or the shinbone, leading to proper diagnosis of rheumatism, damage in the cartilage, damage in the meniscus, etc.

Meanwhile, in a case where the subject moves when a moire image of the subject is captured by the X-ray Talbot imaging device (namely, in a case where there is body motion of the subject), it would be difficult to perform proper reconstruction and generation of a differential phase image, or the like, because of failure in reconstruction of the differential phase image, or of abnormal reconstruction of the differential phase image, even when reconstruction of the captured moire image to generate the differential phase image is attempted.

To cope with this, as described in US Patent No. 5099135, US Patent No. 5485856, JP 2013-180040 A, JP 2013-255536 A, and JP 2014-132977 A, for example, moire image capturing is frequently performed in a state where the posture of the subject is maintained such that no body motion of the subject is generated, by holding the joint section of the subject by a holding unit, or pulling the joint section, or the like, by using a member for position adjustment, when the subject is arranged between the X-ray source (or source grating) and the phase grating, or between the phase grating and the second grating, on the X-ray Talbot imaging device.

Unfortunately, however, when the knee joint section is held by the subject holding member (namely, a holding unit) described in JP 2014-132977 A, for example, the knee joint section might move without using a further fixture to fix the knee joint section held by the subject holding member.

In particular, in a case where the meniscus ME (refer to Fig. 12) is imaged onto a differential phase image, the imaging of the meniscus ME would not be possible until the interface between the meniscus ME and the cartilage of the thighbone TB, or the like, becomes parallel with a radiation emission direction. Therefore, it would not be possible to perform imaging of the meniscus ME onto the differential phase image without properly adjusting an angle of the knee joint section with respect to the radiation emission direction. Unfortunately, however, there is no disclosed mechanism capable of properly adjusting the angle of the knee joint section with respect to the radiation emission direction.

Summary of the Invention

The present invention has been made in view of the above-described problems, and an object of the present invention is to provide an X-ray Talbot imaging device that includes a holding unit capable of properly adjusting the angle of a knee joint section with respect to the radiation emission direction.

To achieve the abovementioned object, according to an aspect, an X-ray Talbot imaging device reflecting one aspect of the present invention comprises: a plurality of gratings including a phase grating; a subject platform configured to hold a subject; a radiation generator configured to emit radiation toward the subject; a radiation flat panel detector configured to capture a moire image; and a holding unit configured to hold a knee joint section as the subject on the subject platform, wherein the holding unit includes: a first holding section configured to hold a proximal side of the joint section; a second holding section configured to hold a distal side of the joint section; a pivot fixing mechanism capable of pivoting the first holding section and the second holding section relative to each other within a plane orthogonal to a grating surface of the phase grating and capable of fixing a relative pivot angle between the first holding section and the second holding section; and an angle adjustment mechanism configured to change an angle of the knee joint section as the subject held by the first holding section and the second holding section, with respect to an emission direction of radiation emitted from the radiation generator.

Brief Description of the Drawings

The above and other objects, advantages and features of the present invention will become more fully understood from the detailed description given hereinbelow and the appended drawings which are given by way of illustration only, and thus are not intended as a definition of the limits of the present invention, and wherein:

Fig. 1 is a view illustrating a principle of a Talbot interferometer;
Fig. 2 is a schematic plan view of a source grating, a phase grating, and a second grating;
Fig. 3 is a schematic diagram of an entire X-ray Talbot imaging device according to the present embodiment;
Fig. 4 is a perspective view of an external appearance of a holding unit according to the present embodiment;
Fig. 5 is a diagram illustrating a vacuum cushion;
Fig. 6 is a diagram illustrating a state where a patient sitting on a chair has inserted his/her thigh and shin into a first holding section and a second holding section, of the holding unit;
Figs. 7A to 7C are diagrams each illustrating a state where a relative pivot angle between the first holding section and the second holding section, on the holding unit, is changed;
Fig. 8 is a diagram illustrating a state where a relative pivot angle θ1 between the first holding section and the second holding section on the holding unit is equal to a knee angle θ2;
Figs. 9A to 9C are diagrams each illustrating a state where an angle of a knee joint section with respect to a radiation emission direction changes when two rails are raised;
Fig. 10 is a photograph illustrating an exemplary absorption image;
Fig. 11 is a photograph of an exemplary differential phase image and an end portion of an articular cartilage; and
Fig. 12 is a diagram illustrating a differential phase image of a knee joint section, in which a meniscus is imaged.

Description of the Preferred Embodiments

Hereinafter, an X-ray Talbot imaging device according to an embodiment of the present invention will be described with reference to the drawings. However, the scope of the invention is not limited to the illustrated examples.

[Principle of X-ray Talbot imaging device]

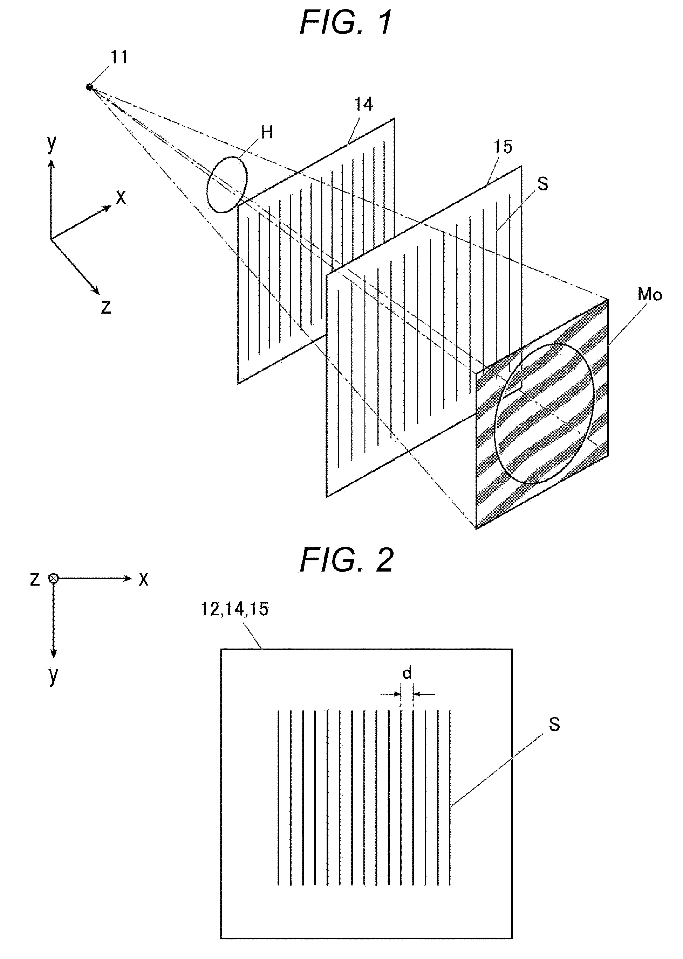

First, a principle of the X-ray Talbot imaging device, namely, a common principle of a Talbot interferometer and a Talbot-Lau interferometer, used in the X-ray Talbot imaging device, will be briefly described with reference to Fig. 1. Note that although Fig. 1 illustrates a case of the Talbot interferometer, similar description can also be fundamentally applied to the Talbot-Lau interferometer.

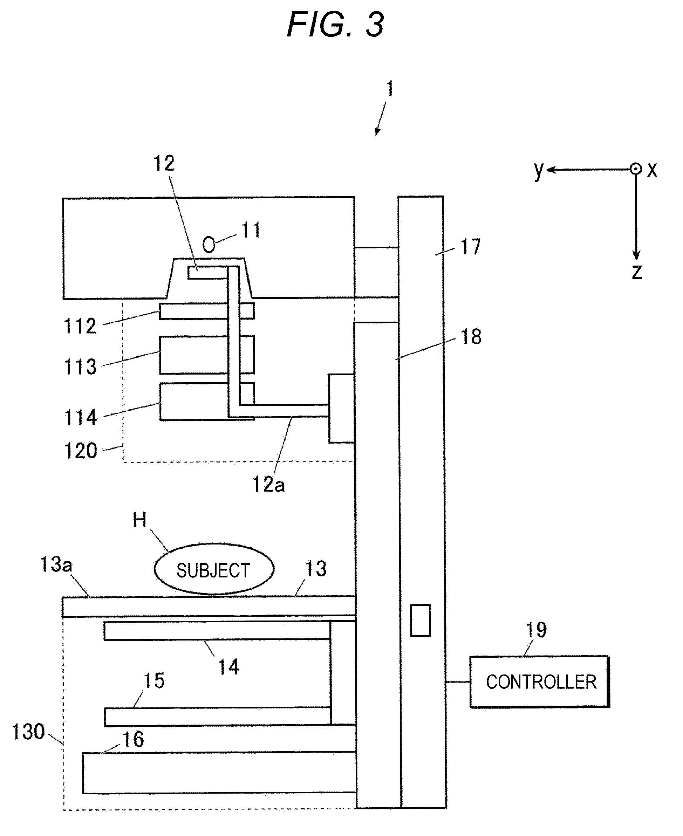

In the Talbot interferometer, a radiation generator 11, a phase grating 14, a second grating 15, and a radiation flat panel detector 16 are arranged sequentially in a radiation emission direction (namely, a z-direction) as illustrated in Fig. 1. In the Talbot-Lau interferometer, a source grating 12 (refer to Figs. 2, 3, and others, to be described below) is arranged near the radiation generator 11, although not illustrated in Fig. 1.

As illustrated in Fig. 2, a plurality of slits S is arranged, at a predetermined interval d, on the phase grating 14 and the second grating 15 (also on the source grating 12 in case of the Talbot-Lau interferometer) in an x-direction orthogonal to the z-direction as a radiation emission direction. The predetermined interval d varies according to the phase grating 14, the second grating 15, and the source grating 12.

When the radiation emitted from the radiation generator 11 (in case of Talbot-Lau interferometer, radiation emitted from the radiation generator 11 and made into multi-light source radiation at the source grating 12) is transmitted through the phase grating 14, the transmitted radiation forms an image with a predetermined distance in the z-direction. This image is referred to as a self-image (also referred to as a grating image, or the like), and a phenomenon in which the self-image is formed in the z-direction with a predetermined distance is referred to as a Talbot effect.

As illustrated in Fig.1, etc., the second grating 15 having the slit S similarly to the phase grating 14 is arranged at a position where the self-image of the phase grating 14 is formed. At this time, the second grating 15 is arranged such that an extending direction (namely, y-axis direction in Fig. 1, etc.) of the slit S of the second grating 15 is slightly angled with respect to the extending direction of the slit S of the phase grating 14. With this arrangement, a moire image Mo formed solely with moire fringes appears on the second grating 15.

Note that Fig. 1 illustrates the moire image Mo separately from the second grating 15 in order to avoid confusion. That is, illustrating the moire image Mo over the second grating 15 might be confusing because the moire fringe and the slit S would be mixed and complicated. In reality, however, the moire image Mo is formed on the second grating 15 or its downstream side.

Meanwhile, when a subject H exists, a phase of radiation is shifted due to the existence of the subject H. This generates, on the moire fringe of the moire image Mo, disturbance having an edge of the subject H as a boundary, leading to generation of the moire image Mo in which disturbance has been generated by the subject H, on the second grating 15 or its downstream side, as illustrated in Fig. 1.

The principle of the Talbot interferometer and the Talbot-Lau interferometer is as described above. It is configured such that the above-described moire image Mo is to be imaged by the radiation flat panel detector 16 (refer to Fig. 3 to be described below) arranged downstream of the second grating 15. The X-ray Talbot imaging device is configured according to the above principle.

[Configuration of X-ray Talbot imaging device]

Hereinafter, an X-ray Talbot imaging device 1 according to the present embodiment, configured based on the above-described principle, will be described. Fig. 3 is a schematic diagram of the entire X-ray Talbot imaging device 1 according to the present embodiment.

The present embodiment describes a case where the X-ray Talbot imaging device 1 is an X-ray Talbot imaging device using a Talbot-Lau interferometer including a source grating (also referred to as G0 grating, multi grating, multi slit, or the like) 12. However, the same description can be applied to an X-ray Talbot imaging device that uses a Talbot interferometer that does not include the source grating 12 but includes only the phase grating 14 and the second grating 15.

In the case described below, as illustrated in Fig. 3, the X-ray Talbot imaging device 1 is configured to emit radiation from the radiation generator 11 provided on an upper side to the subject H in the lower direction. However, the embodiments of the present invention are not limited to this arrangement but can be configured such that radiation is emitted from the radiation generator 11 in a horizontal direction or other arbitrary directions.

As illustrated in Fig. 3, the X-ray Talbot imaging device 1 according to the present embodiment includes the radiation generator 11, the source grating 12, a subject platform 13, the phase grating 14, the second grating 15, the radiation flat panel detector 16, a support column 17, a base stand 18, and a controller 19.

The radiation generator 11 includes, for example, a Coolidge X-ray source and a rotating anode X-ray source, broadly utilized in medical environments. It is also possible to configure such that the device includes another type of radiation source (tube).

In the present embodiment, the source grating 12 is provided downstream side of the radiation generator 11 in the radiation emission direction (namely, z-direction). To avoid transmission of vibration of the radiation generator 11 to the source grating 12, or the like, the source grating 12 in the present embodiment is not attached to the radiation generator 11 but attached to a fixing member 12a mounted on the base stand 18 provided on the support column 17.

On the fixing member 12a, not only the source grating 12, but also a filter (also referred to as an added filter, etc.) 112, a radiation field stop 113, a radiation field lamp 114, and the like, are attached The filter 112 changes radiation quality of the radiation transmitted through the source grating 12. The radiation field stop 113 narrows a radiation field of the emitted radiation. The radiation field lamp 114 is used for positioning by emitting visible rays instead of radiation to the subject H before emitting radiation. Additionally, a first cover unit 120 is arranged around the source grating 12, or the like, in order to protect these components.

On the downstream side of the source grating 12 in the radiation emission direction (namely, z-direction), the subject platform 13 to hold the subject H, the phase grating 14, the second grating 15, the radiation flat panel detector 16, and the like, are provided. At this time, as described above, the distance between the phase grating 14 and the second grating 15 is adjusted such that the second grating 15 can be arranged at a position where the radiation emitted from the radiation generator 11 and transmitted through the phase grating 14 forms a self-image with the predetermined distance from the phase grating 14 in the z-direction.

It is configured such that the radiation flat panel detector 16 is arranged immediately below the second grating 15 and that the moire image Mo generated on the second grating 15 as described above can be imaged by the radiation flat panel detector 16. In addition, in the present embodiment, as illustrated in Fig. 3, a second cover unit 130 is provided for protecting the radiation flat panel detector 16, or the like, specifically for avoiding a leg, or the like, of a patient from hitting or coming in contact with the phase grating 14, the second grating 15, the radiation flat panel detector 16, or the like.

Although not illustrated, the radiation flat panel detector (FPD) 16 is configured to have conversion elements that generate electrical signals in response to the emitted radiation being second-dimensionally (in matrix) arranged and configured to read the electrical signals generated by the conversion elements as image signals. In the present embodiment, the radiation flat panel detector 16 captures the moire image Mo, which is an image of radiation formed on the second grating 15, as an image signal for each of the conversion elements.

In a case where the X-ray Talbot imaging device 1 is configured to capture a plurality of moire images Mo using a fringe scanning method, the plurality of moire images Mo is captured while shifting relative positions of the phase grating 14 and the second grating 15 in the x-axis direction in Figs. 1 and 2 (namely, a direction orthogonal to the extending direction (y-axis direction) of the slit S). Although not illustrated, there is provided a shifting apparatus, or the like, to shift the relative positions of the phase grating 14 and the second grating 15 by the x-axis direction. In a case where the configuration is such that a moire image Mo is imaged by the X-ray Talbot imaging device 1 and that the image is Fourier-transformed to be reconstructed to generate a differential phase image, or the like, there would be no need to provide a shifting apparatus, or the like.

The controller 19 (refer to Fig. 3) in the present embodiment is formed with a computer in which, a central processing unit (CPU), a read only memory (ROM), a random access memory (RAM), an input/output interface, and the like (none of them being illustrated), are connected to a bus. Alternatively, the controller 19 can be configured as a dedicated control apparatus, not as a general-purpose computer as in the present embodiment. Although not illustrated, the controller 19 includes units and apparatuses as appropriate, such as an input unit and a display unit.

The controller 19 is configured to perform overall control over the X-ray Talbot imaging device 1, including, for example, setting a tube voltage, a tube current, and radiation time onto the radiation generator 11. When the X-ray Talbot imaging device 1 is configured to capture the plurality of moire images Mo with the fringe scanning method, the controller 19 controls the above-described shifting apparatus so as to move the phase grating 14 and/or the second grating 15 by a predetermined amount.

Additionally, the controller 19 is configured to perform reconstruction to generate an absorption image (refer to Fig. 10), a differential phase image (refer to Fig. 11), or the like, by oneself, based on one or more moire images Mo transmitted from the radiation flat panel detector 16. Alternatively, the controller 19 is configured to transfer one or more moire images Mo transmitted from the radiation flat panel detector 16 to an external image processing apparatus (not illustrated) and to cause the apparatus to perform reconstruction to generate an absorption image, a differential phase image, or the like.

[Holding unit]

Next, a holding unit 20 according to the present embodiment will be described. The holding unit 20, when in use, is mounted or fixed onto the subject platform 13 of the X-ray Talbot imaging device 1. The holding unit 20 can also be integrally formed with the subject platform 13.

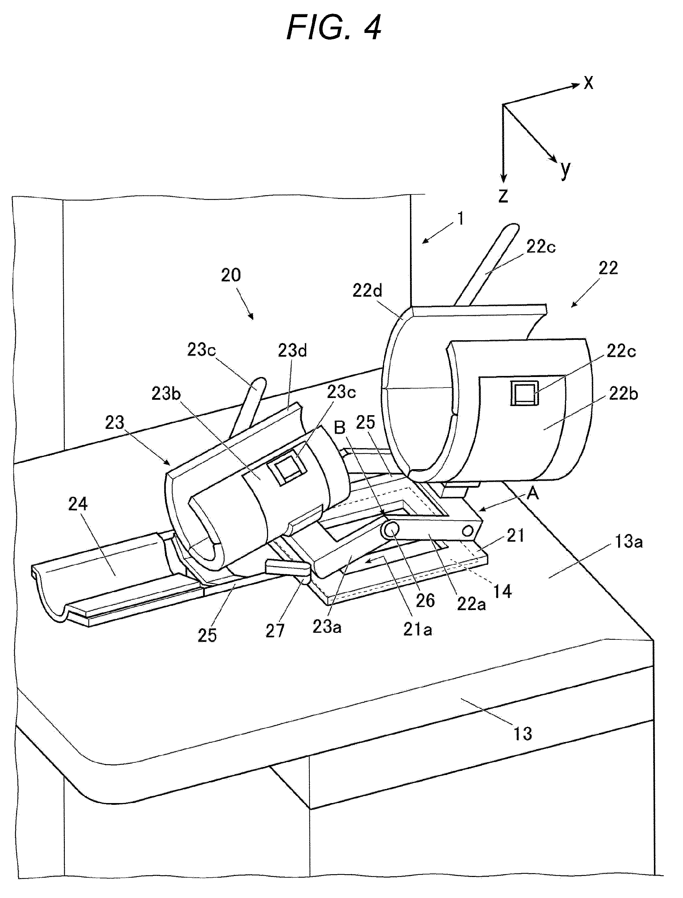

Fig. 4 is a perspective view of an external appearance of the holding unit 20 according to the present embodiment. Hereinafter, directions of up-down and left-right related to the holding unit 20 are applied to the case where the holding unit 20 is arranged as illustrated in Fig. 4. Therefore, when the holding unit 20 is arranged with left and right sides reversed, the left and right in the following description will be naturally reversed.

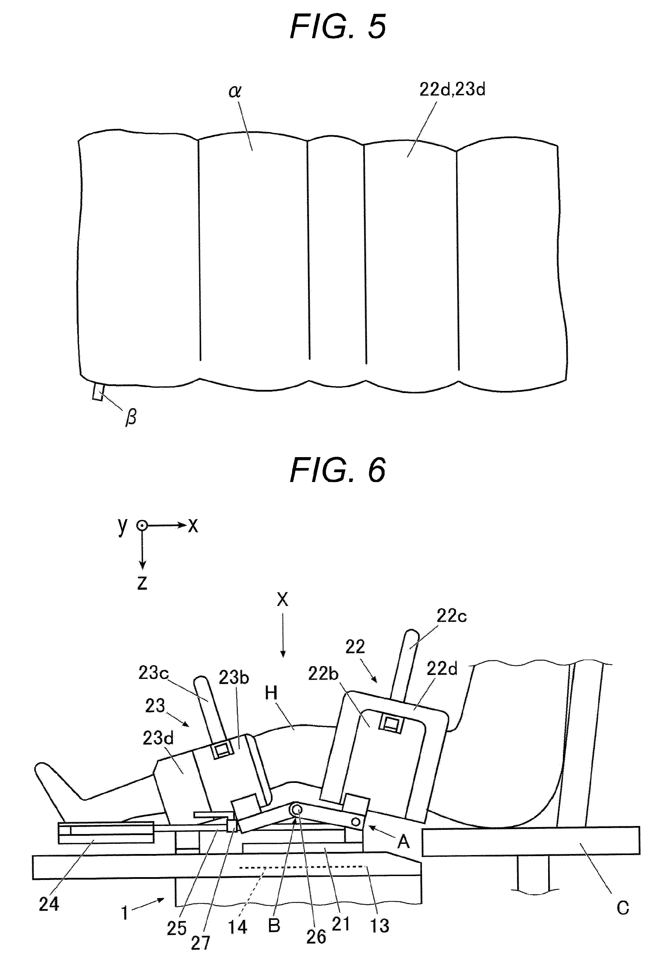

The holding unit 20 is an apparatus to hold a knee joint section as the subject H, on the subject platform 13. In the present embodiment, as illustrated in Fig. 6 to be described below, the knee joint section as the subject H is held on the subject platform 13 such that the patient is sitting on a chair C on a side of the subject platform 13 of the X-ray Talbot imaging device 1, placing both legs on the subject platform 13, the thigh of the target leg for imaging (right leg or left leg) being held by a first holding section 22 of the holding unit 20, and the shin being held by a second holding section 23.

Specifically, as illustrated in Fig. 4, the holding unit 20 includes the first holding section 22 to hold a proximal side (thigh, in this case) of the joint section, and the second holding section 23 to hold a distal side (shin, in this case) of the joint section. In the state illustrated in Fig. 4, the first holding section 22 of the holding unit 20 is arranged on the right side; the second holding section 23 of the holding unit 20 is arranged on the left side, when facing the unit in this direction.

In the present embodiment, the holding unit 20 includes a substantially plate-shaped support section 21 that supports the first holding section 22 and the second holding section 23. On a central portion of the support section 21 of the holding unit 20, there is provided an opening portion 21a, through which radiation emitted from the radiation generator 11 (refer to Fig. 3) toward the subject H is transmitted, to be incident on the phase grating 14 (refer to the broken line in Fig. 4) provided in a lower direction of the opening portion 21a.

In an upper direction of the support section 21 of the holding unit 20, two stick-like rails 25 are provided on front and rear sides of the opening portion 21a of the support section 21, being parallel with each other, extending in a substantially horizontal direction (x-direction). Each of right ends of the two rails 25 is connected to the support section 21 of the holding unit 20 via a hinge structure A. Moreover, between each of left end portions of the two rails 25, rubber or the like is stretched to form a third holding section 24.

With this structure, in the present embodiment, the two rails 25, by moving their left end sides, namely, the third holding section 24 sides in an up-down direction, can be swung in the up-down direction with the right end-side hinge structure A as a fulcrum. This function will be described in detail below.

In the present embodiment, a right end side of a pedestal 22a of the first holding section 22 is connected to the support section 21 of the holding unit 20 via the hinge structure A, similarly to the two rails 25. With this structure, the pedestal 22a of the first holding section 22 can be swung in the up-down direction with the hinge structure A as a fulcrum, similarly to the above-described two rails 25 (although independently from the two rails 25).

On the pedestal 22a of the first holding section 22, a fixture 22b is provided to fasten and fix the thigh of the patient. On the fixture 22b, a fastener 22c is attached so as to fix the thigh of the patient by fastening the fixture 22b wrapping the thigh of the patient from outside.

A left end side of the pedestal 22a of the first holding section 22 is connected to the right end side of a pedestal 23a of the second holding section 23 via a hinge structure B. A left end side of the pedestal 23a of the second holding section 23 is attached to the two rails 25, enabling movement along the two rails 25 without separating from the two rails 25.

On the pedestal 23a of the second holding section 23, a fixture 23b is provided to fasten and fix the shin of the patient. On the fixture 23b, a fastener 23c is attached so as to fix the shin of the patient by fastening the fixture 23b that wraps the shin of the patient from outside.

In the present embodiment, a screw 26 is provided at a portion where the hinge structure B exists. By screw-fastening the hinge structure B using the screw 26, it is not possible to perform relative pivot operation of the pedestal 22a of the first holding section 22 and the pedestal 23a of the second holding section 23 via the hinge structure B. By releasing screw-fastening of the hinge structure B using the screw 26, it is possible to perform relative pivot operation of the pedestal 22a of the first holding section 22 and the pedestal 23a of the second holding section 23 via the hinge structure B. This function will be described in detail below.

In the present embodiment, vacuum cushions 22d and 23d are respectively arranged inside the fixtures 22b and 23b of the first holding section 22 and the second holding section 23. Note that the vacuum cushions 22d and 23d are not necessarily required. In the present embodiment, each of the vacuum cushions 22d and 23d has a role to reinforce a function of each of the fixtures 22b and 23b of the first holding section 22 and the second holding section 23 to fix the thigh and the shin of the patient.

Each of the vacuum cushions 22d and 23d has a structure as illustrated in Fig. 5, for example. Specifically, each of the vacuum cushions 22d and 23d has a substantially rectangular shape and is formed with a plurality of hollow bag-like members $\alpha$ being linked with each other. Each of the bag-like members $\alpha$ has partial internal connection to an adjacent bag-like member $\alpha$, with internal air of the bag-like member $\alpha$ being movable to the inside of the adjacent bag-like member $\alpha$.

Although not illustrated, each of the bag-like members $\alpha$ of the vacuum cushions 22d and 23d includes a large number of round-shaped globules formed of resin or the like, including foamed urethane, and each of the globules inside the bag-like member $\alpha$ is not allowed to move to an adjacent bag-like member $\alpha$. Each of the globules can freely move inside the bag-like member $\alpha$ when there is air inside the bag-like member $\alpha$. When the air of the bag-like member $\alpha$ is removed, however, there is no space where the globules can move inside the bag-like member $\alpha$, leading to immobility of the globules inside the bag-like member $\alpha$. With this mechanism, each of the bag-like member $\alpha$ cannot be easily deformed.

Accordingly, in a state where the thigh and the shin of the patient are inserted into the vacuum cushions 22d and 23d inside each of the fixtures 22b and 23b of the first holding section 22 and the second holding section 23, namely, in a state where the thigh or the shin of the patient is wrapped by the vacuum cushions 22d and 23d, removing air from each of the bag-like members $\alpha$ by sucking air inside the vacuum cushions 22d and 23d from a suction port $\beta$ using a suction pump, etc. would create a state where the vacuum cushions 22d and 23d wrap and hold the thigh and the thin of the patient such that the portions are not to be easily deformed.

In this manner, since use of the vacuum cushions 22d and 23d could fix the thigh and the shin of the patient with the vacuum cushions 22d and 23d, it is possible with the vacuum cushions 22d and 23d to reinforce a function to fix the thigh and the shin of the patient using the fixtures 22b and 23b of the first holding section 22 and the second holding section 23.

Meanwhile, as described above, in the present embodiment, the third holding section 24 is formed by stretched rubber between left end portions of the two rails 25, making it possible to properly hold the heel of the patient placed on the third holding section 24. That is, the holding unit 20 according to the present embodiment is provided with the third holding section 24 that holds the heel of the patient, namely, a portion on a further distal side of the distal-side of the knee joint section (namely, shin) held by the second holding section 23.

As described above, by not only holding the thigh and the shin of the patient with the first holding section 22 and the second holding section 23 but also holding the heel portion of the patient with the third holding section 24, it is possible to fix the foot of the patient firmly in place and to more properly prevent occurrence of body motion.

[Action]

Next, actions of the X-ray Talbot imaging device 1 according to the present embodiment, particularly actions of the holding unit 20, will be described.

When the knee joint section of the patient as the subject H is imaged, the patient sits on the chair C arranged on the side of the X-ray Talbot imaging device 1 before imaging. In this state, the chair C is raised and the orientation of the chair C is changed so as to face the subject platform 13 of the X-ray Talbot imaging device 1. As illustrated in Fig. 6, the thigh is inserted into the first holding section 22, the shin is inserted into the second holding section 23, and the heel is inserted into the third holding section 24 such that the knee joint section (namely, the subject H) of a target leg (right leg or left leg) for imaging, of the patient is positioned above the opening portion 21a (refer to Fig. 4) of the support section 21 of the holding unit 20.

Under this condition, a knee angle $\theta 2$ of the patient is adjusted. That is, as described above, while the thigh of the patient is inserted into the first holding section 22 and the shin of the patient is inserted into the second holding section 23, the knee angle of the patient is adjusted by relatively pivoting the first holding section 22 and the second holding section 23 via the hinge structure B of the holding unit 20.

Specifically, as described above, the first holding section 22 of the holding unit 20 can be swung in the up-down direction with the hinge structure A as a fulcrum. In other words, it is possible to pivot the first holding section 22 within a plane (xz plane) orthogonal to a grating surface of the phase grating 14 (refer to Figs. 4 and 6).

The second holding section 23 of the holding unit 20 is configured such that the right end side of the pedestal 23a is connected to the left end side of the pedestal 22a of the first holding section 22 via the hinge structure B, and thus, the left end side of the pedestal 23a of the second holding section 23 moves along the two rails 25 without separating from the two rails 25. In other words, it is also possible to pivot the second holding section 23 of the holding unit 20 within the plane (xz plane) orthogonal to the grating surface of the phase grating 14.

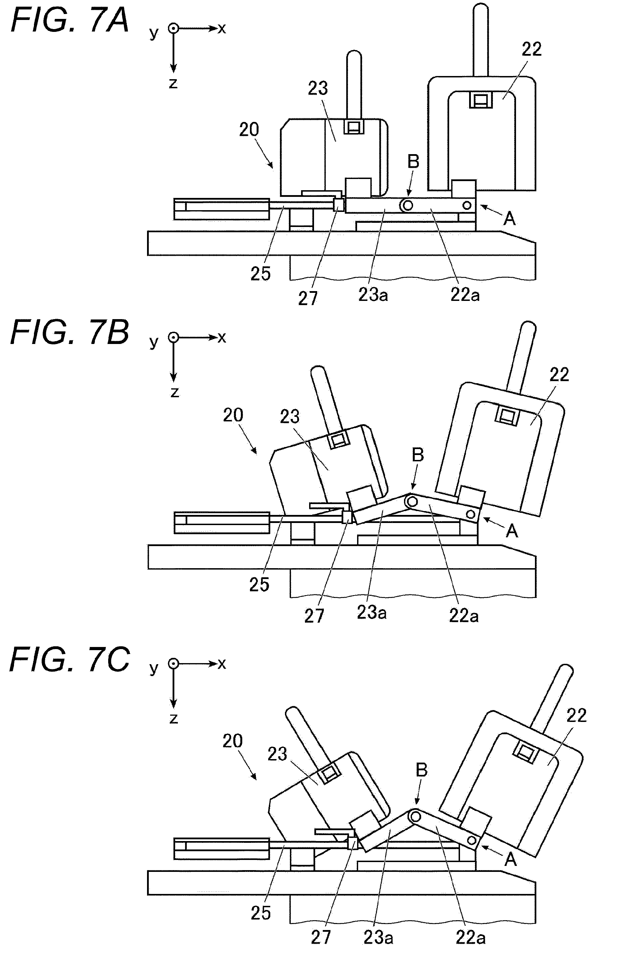

As illustrated in Figs. 7A to 7C, when the first holding section 22 of the holding unit 20 is swung with the hinge structure A as a fulcrum, such that the pedestal 22a moves upward, the second holding section 23 is swung while the right end side of the pedestal 23a is raised via the hinge structure B. Specifically, when the first holding section 22 is pivoted clockwise within the xz plane, the second holding section 23 pivots counterclockwise within the xz plane. That is, the first holding section 22 and the second holding section 23 pivot relative to each other within the xz plane (namely, a plane orthogonal to the grating surface of the phase grating 14).

Note that in this case a relative pivot angle $\theta 1$ between the first holding section 22 and the second holding section 23 increases. Additionally, the left end side (side moving along the rail 25) of the pedestal 23a of the second holding section 23 moves in the right direction (direction to approach the first holding section 22) as the pivot angle $\theta 1$ increases.

In contrast, in a state of Fig. 7C, when the first holding section 22 of the holding unit 20 pivots counterclockwise within the xz plane, the second holding section 23 pivots clockwise within the xz plane, so as to be in the state illustrated in Figs. 7B and 7A. In this case, the relative pivot angle $\theta 1$ between the first holding section 22 and the second holding section 23 decreases, and the left end side (side moving along the rail 25) of the pedestal 23a of the second holding section 23 moves in the left direction (direction away from the first holding section 22) as the pivot angle $\theta 1$ decreases.

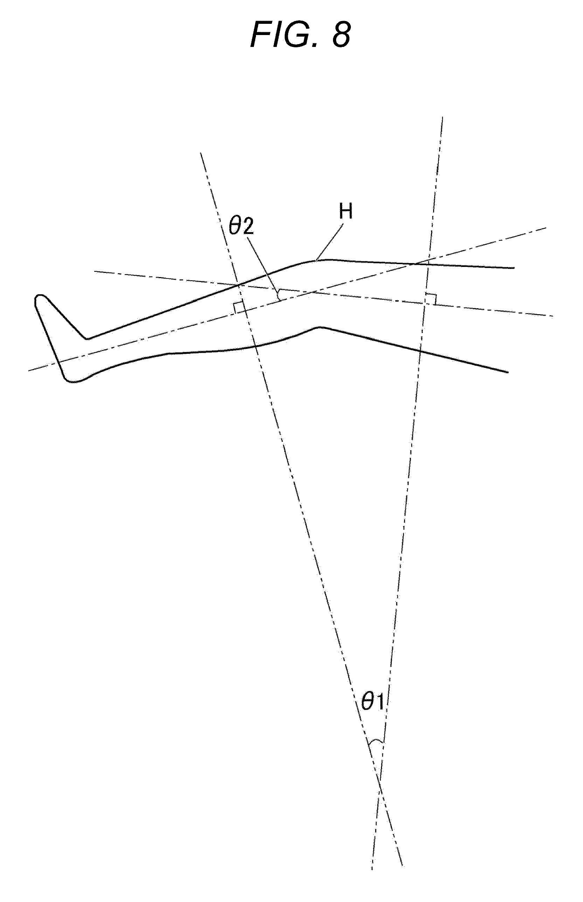

As illustrated in Fig. 8, on the holding unit 20, the relative pivot angle $\theta 1$ between the first holding section 22 and the second holding section 23 is equal to the knee angle $\theta 2$ of the patient. Therefore, in the holding unit 20 according to the present embodiment, by swinging as described above the first holding section 22 in the up-down direction with the hinge structure A as a fulcrum (in practice, moving the hinge structure B portion in the up-down direction) so as to pivot the first holding section 22 and the second holding section 23 relative to each other within the xz plane (plane orthogonal to the grating surface of the phase grating 14), it is possible to adjust the knee angle $\theta 2$ of the patient.

Subsequently, in the state where the knee angle $\theta 2$ of the patient has been properly adjusted by pivoting the first holding section 22 and the second holding section 23 relative to each other, the hinge structure B portion is screwed by fastening the screw 26 at the hinge structure B portion, so as to fix the knee angle $\theta 2$ adjusted as described above (namely, relative pivot angle $\theta 1$ between the first holding section 22 and the second holding section 23).

In this manner, in the present embodiment, it is configured such that mainly the hinge structure B and the screw 26 enable relative pivoting between the first holding section 22 and the second holding section 23 of the holding unit 20 within the xz plane (plane orthogonal to the grating surface of the phase grating 14), and function as a pivot fixing mechanism capable of fixing the relative pivot angle θ1 between the first holding section 22 and the second holding section 23.

Alternatively, instead of, or in addition to screwing the hinge structure B with the screw 26, it would be possible to configure such that the left end side of the pedestal 23a of the second holding section 23 that moves along the rail 25 together with the relative pivoting between the first holding section 22 and the second holding section 23 is fixed to the rail 25 using a stopper 27 (refer to Figs. 4, 6, and 7A to 7C) so as not to allow the left end side of the pedestal 23a of the second holding section 23 to move along the rail 25, and that the relative pivot angle θ1 between the first holding section 22 and the second holding section 23 is fixed.

After the knee angle θ2 has been adjusted to be fixed as described above, air inside the vacuum cushions 22d and 23d is removed in a state where the vacuum cushions 22d and 23d wrap the thigh and the shin of the patient, respectively, so as to fix the thigh and the shin of the patient with the vacuum cushions 22d and 23d.

Thereafter, the thigh and the shin of the patient are wrapped with the fixtures 22b and 23b over the vacuum cushions 22d and 23d, and then, the fixtures 22b and 23b are respectively fastened with the fasteners 22c and 23c, so as to fasten and fix the thigh and the shin of the patient.

In this state, however, although the knee angle θ2 of the patient is adjusted and fixed, the angle of the knee joint section with respect to the radiation emission direction (z-direction) has not been adjusted. To cope with this, the present embodiment is configured to adjust the angle of the knee joint section with respect to the radiation emission direction (z-direction) by swinging the two rails 25 in the up-down direction with the hinge structure A as a fulcrum.

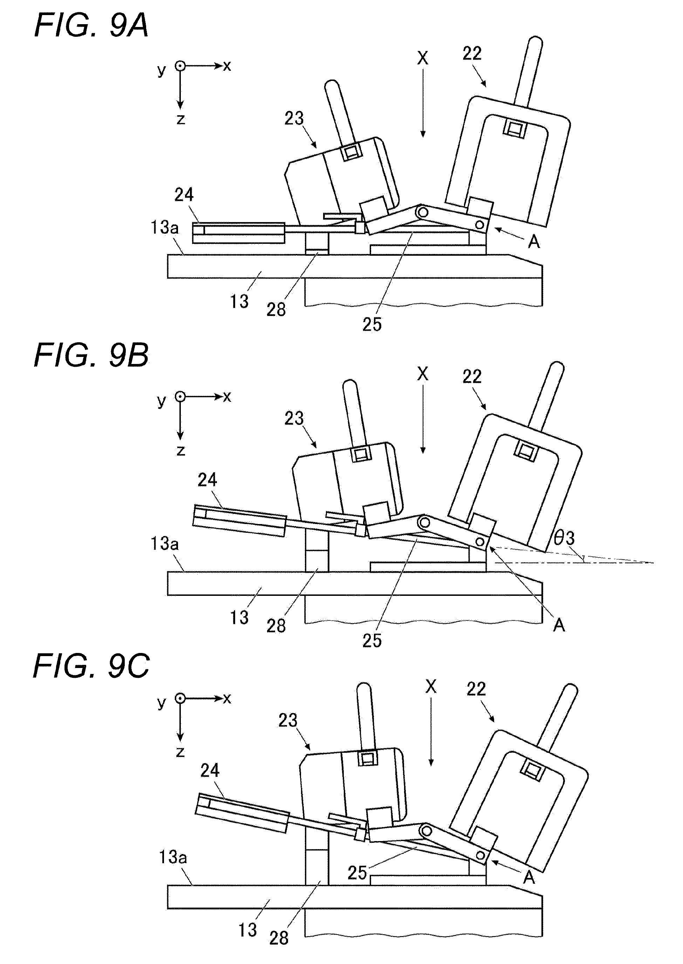

In other words, in the holding unit 20 according to the present embodiment, it is possible, as described above, to allow the two rails 25 to change the angle of the first holding section 22 and the second holding section 23 to which the pivot angle θ1 has been fixed as described above, and the angle of the knee joint section (not illustrated) held by the holding sections, with respect to the radiation emission direction (z-direction), as described in Figs. 9A to 9C, by moving the left end side, namely, the third holding section 24-side of the rails 25 in the up-down direction.

On this occasion, when it is assumed, as illustrated in Fig. 9A, that the angle Θ of the knee joint section with respect to the radiation emission direction (z-direction) in a state where the two rails 25 are not raised (namely, the two rails 25 are extending horizontally) is 90°, as illustrated in Fig. 9B, the angle Θ of the knee joint section with respect to the radiation emission direction (z-direction) in a case where the two rails 25 are raised by an angle θ3 from the horizontal direction would be

$$\Theta = 90° - \theta3 \quad ... \quad (1).$$

In this manner, it is possible, in the present embodiment, to properly adjust the angle Θ of the knee joint section with respect to the radiation emission direction (z-direction). By achieving, in this manner, proper adjustment of the angle Θ of the knee joint section with respect to the radiation emission direction (z-direction), it is possible, for example in imaging a meniscus of the knee, to properly adjust the angle Θ of the knee joint section such that an interface between the meniscus and the cartilage of the thighbone, etc. becomes parallel with the radiation emission direction. It is also possible, as illustrated in Fig. 12, to properly image the meniscus ME (more specifically, the interface between the meniscus ME and the cartilage of the thighbone TB, etc.) in the differential phase image reconstructed from the captured moire image Mo.

Furthermore, in the present embodiment, mainly the hinge structure A and the two rails 25, as described above, function as an angle adjustment mechanism to change the angle Θ of the knee joint section as the subject H, held by the first holding section 22 and the second holding section 23 of the holding unit 20, with respect to the radiation emission direction (z-direction) of the radiation emitted from the radiation generator 11 (refer to Fig. 3, etc.).

Moreover, in the present embodiment, it is configured such that the angle Θ of the knee joint section with respect to the radiation emission direction (z-direction) is changed by changing the angle θ3 of each of the first holding section 22 and the second holding section 23 from the horizontal direction, while changing a height of the left side (namely, second holding section 23-side) of the rail 25 from the subject platform 13 from the surface 13a by raising the left side of the rail 25 using, for example, an actuator 28, with the hinge structure A on the right end side of the rail 25 as a fulcrum, as described above.

With this configuration, it is possible to properly and easily change the angle Θ of the knee joint section with respect to the radiation emission direction (z-direction) and also to properly change the angle Θ with a simple configuration.

In the present embodiment, the configuration is such that, as described above, the height of the left end side (namely, the second holding section 23-side) of the rail 25 from the surface 13a of the subject platform 13 is changed with the right end side (namely, the first holding section 22-side) of the rail 25 as a fulcrum. Alternatively, it is possible to configure such that, in contrast to this, the height of the right side (namely, the first holding section 22-side) of the rail 25 from the surface 13a of the subject platform 13 is changed with the left end side (namely, the second holding section 23-side) of the rail 25 as a fulcrum. Further alternatively, it is possible to configure such that the heights of both sides (namely, the first holding section 22-side and the second holding section 23-side) of the rail 25 from the surface 13a of the subject platform 13 are each changed.

Furthermore, when the heights of the first holding section 22 and the second holding section 23 of the holding unit 20 configured to hold the knee joint section as the subject, from the surface 13a of the subject platform 13 are changed, as in the present embodiment, it is possible to configure, at the same time, such that the height of the third holding section 24 that holds the heel of the patient, from the surface 13a of the subject platform 13 is changed. With this configuration, it is possible to maintain a state where the third holding section 24 holds the heel of the patient even when the height of the first holding section 22 or the second holding section 23 is changed as described above. Accordingly, by configuring such that the third holding section 24 holds the heel of the patient, it is possible to fix the foot of the patient firmly in place. This would be preferable because it is possible to properly maintain the functions of the third holding section 24 to properly prevent occurrence of body motion.

To achieve this, it would be preferable that the holding unit 20 includes a third holding section height adjustment mechanism to change the height of the third holding section 24 from the surface 13a of the subject platform 13. To cope with this, in the present embodiment, the third holding section 24 is provided at the left end portion of the two rails 25, and thus, the height of the third holding section 24 from the surface 13a of the subject platform 13 can be properly changed because the third holding section 24 swings with the hinge structure A as a fulcrum, together with the two rails 25.

In this regard, in the present embodiment, mainly the hinge structure A and the two rails 25 function as the third holding section height adjustment mechanism to change the height of the third holding section 24 of the holding unit 20 from the surface 13a of the subject platform 13.

Furthermore, when the first holding section 22 and the second holding section 23 of the holding unit 20 are relatively pivoted within the xz plane (namely, a plane orthogonal to the grating surface of the phase grating 14), as described above, or when the angle Θ of the knee joint section as the subject H with respect to the radiation emission direction (z-direction) is changed by swinging the two rails 25 in the up-down direction with the hinge structure A as a fulcrum, it might be possible that posture of the patient sitting on the chair C (refer to Fig. 6) becomes unstable, or the patient feels tightness.

To avoid this, it is preferable to enable adjustment of height of the chair C from the floor surface. In other words, it would be preferable to provide a proximal-side height adjustment mechanism (in this case, height-adjustable chair C) to adjust the height of a further proximal-side portion (namely, buttocks of the patient) than the proximal side (namely, thigh) of the knee joint section held by the first holding section 22 of the holding unit 20.

[Effects]

As described above, by utilizing the X-ray Talbot imaging device 1 according to the present embodiment, it is possible to pivot the first holding section 22 and the second holding section 23 relative to each other within the xz plane (namely, a plane orthogonal to the grating surface of the phase grating 14) and to fix them properly at the proper pivot angle θ1 (namely, to fix the knee joint section at the angle θ2 equal to the pivot angle θ1), by using a pivot fixing mechanism (namely, the hinge structure B or the screw 26) of the holding unit 20. Furthermore, it is possible to change the angle Θ of the knee joint section as the subject H held by the first holding section 22 and the second holding section 23, with respect to the radiation emission direction (z-direction) by using the angle adjustment mechanism (namely, the hinge structure A and the two rails 25).

Therefore, according to the X-ray Talbot imaging device 1 in the present embodiment, it is possible to properly hold the knee joint section as the subject H by the first holding section 22 and the second holding section 23, and to properly adjust the angle Θ of the knee joint section with respect to the radiation emission direction (z-direction).

Accordingly, it is possible, for example in imaging a meniscus of the knee, to properly adjust the angle Θ of the knee joint section such that the interface between the meniscus and the cartilage of the thighbone, etc. becomes parallel with the radiation emission direction. It is also possible, as illustrated in Fig. 12, to properly image the meniscus ME (more specifically, interface between the meniscus ME and the cartilage of the thighbone TB, etc.) in the differential phase image reconstructed from the captured moire image Mo.

It is naturally understandable that the present invention is not limited to the above-described embodiments and can be modified as appropriate within the spirit and scope of the present invention.

Although the present invention has been described and illustrated in detail, it is clearly understood that the same is by way of illustrated and example only and is not to be taken by way of limitation, the scope of the present invention being interpreted by terms of the appended claims.

**Claims**

1. An X-ray Talbot imaging device (1), **characterized by** comprising:

   a plurality of gratings including a phase grating (14);
   a subject platform (13) configured to hold a subject (H) ;
   a radiation generator (11) configured to emit radiation toward the subject (H);

a radiation flat panel detector (16) configured to capture a moire image (Mo); and
a holding unit (20) configured to hold a knee joint section as the subject (H) on the subject platform (13),
wherein the holding unit (20) includes:

a first holding section (22) configured to hold a proximal side of the joint section;
a second holding section (23) configured to hold a distal side of the joint section;
a pivot fixing mechanism (B, 26) capable of pivoting the first holding section (22) and the second holding section (23) relative to each other within a plane orthogonal to a grating surface of the phase grating (14) and capable of fixing a relative pivot angle ($\theta$1) between the first holding section (22) and the second holding section (23); and
an angle adjustment mechanism (A, 25) configured to change an angle ($\Theta$) of the knee joint section as the subject (H) held by the first holding section (22) and the second holding section (23), with respect to an emission direction of radiation emitted from the radiation generator (11).

2. The X-ray Talbot imaging device (1) according to claim 1, **characterized in that** the angle adjustment mechanism (A, 25) is configured to change the angle ($\Theta$) by changing a height of the first holding section (22) side from a surface of the subject platform (13), a height of the second holding section (23) side from the surface of the subject platform (13), or heights of the first holding section (22) side and the second holding section (23) side from the surface of the subject platform (13), among the first holding section (22) and the second holding section (23) configured to hold the knee joint section as the subject (H).

3. The X-ray Talbot imaging device (1) according to claim 1 or 2,
**characterized in that** the holding unit (20) further includes a third holding section (24) configured to hold a further distal-side portion than the distal side of the joint section held by the second holding section (23).

4. The X-ray Talbot imaging device (1) according to claim 3,
**characterized in that** the holding unit (20) includes a third holding section height adjustment mechanism (A, 25) configured to change a height of the third holding section (24) from the surface of the subject platform (13).

5. The X-ray Talbot imaging device (1) according to any one of claims 1 to 4, **characterized by** comprising a proximal-side height adjustment mechanism (C) configured to adjust a height of a further proximal-side portion than the proximal side of the joint section held by the first holding section (22).

# FIG. 1

# FIG. 2

# FIG. 3

CONTROLLER

# FIG. 4

## FIG. 5

## FIG. 6

*FIG. 7A*

20

23

22

B

A

25

27    23a    22a

*FIG. 7B*

20

23

22

B

A

25

27    23a    22a

*FIG. 7C*

20

23

22

B

A

25

27    23a    22a

# FIG. 8

## FIG. 9A

## FIG. 9B

## FIG. 9C

*FIG. 10*

*FIG. 11*

# FIG. 12

Europäisches
Patentamt

European
Patent Office

Office européen
des brevets

# EUROPEAN SEARCH REPORT

Application Number

EP 16 17 1863

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JP 2013 255536 A (KONICA MINOLTA INC) 26 December 2013 (2013-12-26) * paragraph [0087] - paragraph [0092]; figures 39-41 * | 1-5 | INV. A61B6/04 A61B6/00 A61F5/37 |
| A | JP 2011 101662 A (OSAKAFU KEISATSU KYOKAI OSAKA KEISATSU BYOIN) 26 May 2011 (2011-05-26) * paragraph [0004] - paragraph [0007]; figures 1-3 * * paragraph [0022] - paragraph [0028] * | 1-5 | |
| A | WO 2015/015851 A1 (KONICA MINOLTA INC [JP]) 5 February 2015 (2015-02-05) * paragraph [0184] - paragraph [0201]; figures 1,25-27 * * paragraph [0016] * | 1-5 | |

|  |
|---|
| TECHNICAL FIELDS SEARCHED (IPC) |
| A61B A61F |

The present search report has been drawn up for all claims

| Place of search | Date of completion of the search | Examiner |
|---|---|---|
| Munich | 19 July 2016 | Martinez Möller, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 17 1863

This annex lists the patent family members relating to the patent documents cited in  the above-mentioned European search report.
The members are as contained in the European Patent Office EDP file on
The European Patent Office is in no way liable for these particulars which are merely given for the purpose of information.

19-07-2016

| Patent document cited in search report | | Publication date | Patent family member(s) | Publication date |
|---|---|---|---|---|
| JP 2013255536 | A | 26-12-2013 | NONE | |
| JP 2011101662 | A | 26-05-2011 | NONE | |
| WO 2015015851 | A1 | 05-02-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5812629 A
- US 5099135 A
- US 5485856 A
- JP 2013180040 A
- JP 2013255536 A
- JP 2014132977 A

**Non-patent literature cited in the description**

- **JUNJI TANAKA et al.** Cadaveric and in vivo human joint imaging based on differential phase contrast by X-ray Talbot-Lau interferometry. *Z. Med. Phys.,* 2013, vol. 23, 222-227